# EUROPEAN PATENT APPLICATION

(11) **EP 4 834 868 A1**
(43) Date of publication of application: **04.11.2026**
(21) Application number: 25305605.5
(22) Date of filing: 28.04.2025
(51) Int. Cl.: C07C 4/04, C07C 11/04, C07C 7/04, C07C 11/06, C07C 9/08, C07C 7/00, C10G 11/00

(54) **ETHYLENE PRODUCTION METHOD**

(71) Applicant: Technip Energies France, 92741 Nanterre Cedex (FR)
(72) Inventor: LOEFFLER, Louis, 92741 Nanterre Cedex (FR); SLACK, Steven Michael, 92741 Nanterre Cedex (FR); HOLLAND, Adrian Charles, 92741 Nanterre Cedex (FR); MALATY, Wadie, 92741 Nanterre Cedex (FR)
(74) Representative: Edson, Russell Gregory

(57) **Abstract**

Provided is an ethylene production method, wherein the method comprises a) cracking a feedstock to convert it into a product stream comprising ethylene, propylene and propane; b) separating the product stream into an ethylene enriched stream and a propylene enriched stream comprising propane; c) separating the propylene enriched stream to form a top product enriched in propylene and a bottoms product enriched in propane and further comprising a green oil impurity; and d) separating the propane from the green oil impurity in the bottoms product by fractionation in a heavies removal unit to produce a bottoms product enriched in green oil and a top product enriched in propane.

## Description

### Field

There is provided an ethylene production method which includes a step of removing green oil impurities and a system for performing said ethylene production method.

### Background

Ethylene is one of the most important petrochemical intermediates and is a feedstock for a variety of chemicals and products for example polyethylene, often used for food packaging, houseware and toys, ethylene glycols, often used in antifreeze and ethylene oxide, often used in carpets and fabrics.

One of the main methods for producing ethylene involves cracking a hydrocarbon-containing feedstock to produce a stream comprising ethylene and propylene, as well as propane and other olefins. A common byproduct of this cracking process is methyl acetylene/propadiene (MAPD). MAPD compounds are often converted to propylene and propane by hydrogenation, but this process can generate impurities such as green oil, which is typically a mixture of C4-C20 oligomers and/or unsaturated hydrocarbons.

Green oil formation and fouling is known to lead to operational difficulties in ethylene production plants such as reduced furnace run length, reduced furnace efficiency, and catalyst deactivation. In ethylene production plants, propane is often recycled for further cracking, however the green oil contained in the stream can impact furnace run length and efficiency if it is reintroduced to the cracking furnaces with the propane stream.

One method of removing green oil generated by selective hydrogenation is to fractionate the C4+ green oil compounds from the MAPD compounds contained in the C3 hydrocarbon cut of propylene rich streams. US6414205B1 describes a process in which MAPD is reacted with hydrogen to produce propylene in a single pass fixed bed reactor and then again in a reactive distillation column containing a hydrogenation catalyst.

However, packed catalytic columns have several drawbacks including potential for thermal gradients leading to hot spots, difficulty in maintaining consistent temperature control, and challenges in scaling up the reactor for industrial applications, as well as the financial implications of catalyst regeneration.

Other methods for removing green oil in ethylene plants include the use of a wash tower to effectively wash the green oil created from C2 acetylene conversion from an ethylene stream. However, this method can only be utilised for vapour-phase streams and not the removal of green oil from liquid-phase streams. Further, these methods can be expensive and inefficient depending on operating conditions.

As such a more efficient method for removing green oil impurities from liquid-phase streams is required.

### Summary

There is provided an ethylene production method, wherein the method comprises:
a) cracking a feedstock to convert it into a product stream comprising ethylene, propylene and propane;
b) separating the product stream into an ethylene enriched stream and a propylene enriched stream comprising propane;
c) separating the propylene enriched stream to form a top product enriched in propylene and a bottoms product enriched in propane and further comprising a green oil impurity; and
d) separating the propane from the green oil impurity in the bottoms product by fractionation in a heavies removal unit to produce a bottoms product enriched in green oil and a top product enriched in propane.

One or more embodiments include the method of the previous paragraph, wherein the fractionation in the heavies removal unit comprises a step of vaporising the propane using a reboiler exchanger, optionally utilizing steam as a heating medium at a pressure of about 150 kPa(g) to about 250 kPa(g), and/or about 100°C to about 150°C.

One or more embodiments include the method of any previous paragraph, further comprising controlling vaporisation of the propane by controlling and/or adjusting the ratio of the amount of top product enriched in propane produced in the heavies removal unit to the amount of bottoms product enriched in propane and further comprising the green oil impurity that is fed to the heavies removal unit, optionally wherein the controlling is performed by a ratio controller.

One or more embodiments include the method of any previous paragraph, further comprising monitoring the temperature of a reboiler outlet stream and adjusting the ratio of the top product to the bottoms product when the reboiler outlet stream temperature exceeds a temperature threshold, optionally wherein the adjustments is performed by a reboiler outlet temperature controller.

One or more embodiments include the method of any previous paragraph, wherein the heavies removal unit has an inlet pressure of from about 1000 kPa(g) to about 1500 kPa(g), optionally about 1000 kPa(g) to about 1300 kPa(g).

One or more embodiments include the method of any previous paragraph, wherein the heavies removal unit has an inlet temperature of from about 25°C to about 90°C, optionally from about 35°C to about 75°C, optionally from about 40°C to about 50°C.

One or more embodiments include the method of any previous paragraph, wherein the green oil impurity is present in the bottoms product enriched in propane and further comprising a green oil impurity in an amount of at least about 2% by weight.

One or more embodiments include the method of any previous paragraph, wherein the top product enriched in propane contains green oil impurity in an amount of less than about 1% by weight.

One or more embodiments include the method of any previous paragraph, wherein the green oil impurity is continuously removed from the heavies removal unit.

One or more embodiments include the method of any previous paragraph, further comprising the step of recycling the top product enriched in propane for cracking.

One or more embodiments include the method of any previous paragraph, wherein the heavies removal unit comprises a packed bed column and/or is catalyst-free.

One or more embodiments include the method of any previous paragraph, wherein the bottoms product enriched in propane and further comprising a green oil impurity is in the liquid phase.

One or more embodiments include the method of any previous paragraph, further comprising the step of recovering the bottoms product enriched in green oil and mixing it with gasoline and/or oil product streams.

Provided herein is a system for producing ethylene, wherein the system comprises:
a reactor for cracking a feedstock to convert it into a product stream comprising ethylene, propylene and propane;
a separation unit for separating the product stream into an ethylene enriched stream and a propylene enriched stream which further comprises propane;
a C3 splitting unit for separating the propylene enriched stream to form a top product enriched in propylene and a bottoms product enriched in propane and further comprising a green oil impurity; and
a heavies removal unit for separating the propane from the green oil impurity in the bottoms product by fractionation to produce a bottoms product enriched in green oil and a top product enriched in propane.

One or more embodiments include the system of the previous paragraph, wherein the heavies removal unit comprises a packed bed column.

One or more embodiments include the system of any previous paragraph, wherein the heavies removal unit is catalyst-free.

One or more embodiments include the system of any previous paragraph, further comprising a reboiler exchanger configured to transfer heat to the heavies removal unit.

One or more embodiments include the system of any previous paragraph, wherein the system comprises a ratio controller between the heavies removal unit product and C3 splitting unit, optionally wherein the ratio controller is configured to adjust the ratio of the amount of top product enriched in propane produced in the heavies removal unit to the amount of bottoms product enriched in propane and further comprising the green oil impurity that is fed to the heavies removal unit.

One or more embodiments include the system of any previous paragraph, further comprising an outlet temperature controller between the reboiler exchanger and the heavies removal unit, optionally wherein the outlet temperature controller is configured to adjust the ratio of the top product to the bottoms product when the reboiler outlet stream temperature is determined to exceed a temperature threshold.

Also provided herein is a method of removing a green oil impurity from a propane stream produced in an ethylene production method, wherein the method comprises:
separating the propane in the propane stream from the green oil impurity by fractionation in a heavies removal unit to produce a bottoms product enriched in green oil and a top product enriched in propane.

The process and system provided herein utilises fractionation to remove green oil impurities downstream of propylene production. This enables propane to be recycled to the cracking furnaces in the absences of green oil impurities, minimising the risk of green oil fouling in the cracking furnaces from the recycle propane stream. In addition, compared to known methods, the requirement for additional hydrogenation catalysts or catalytic columns is eliminated. As such the costs in terms of plant downtime associated with catalyst regeneration as well as the financial implications of supplying and maintaining hydrogenation catalysts are significantly reduced. The risk of green oil impurities fouling or poisoning catalytic beds is also minimised.

The fractionation used in the process provided herein also advantageously provides a method for removing green oil from liquid streams.

### Brief Description of the Drawings

Embodiments of the method and system will now be described in detail, by way of example only, with reference to the figures.

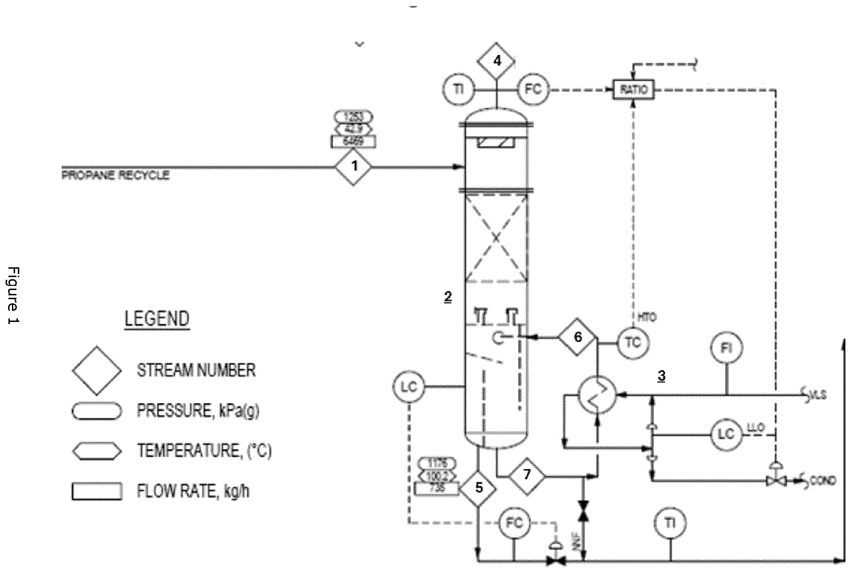

Figure 1 shows a schematic of part of an exemplary ethylene production system embodiment.

### Detailed Description

For the purposes of this description, green oil is generally assumed to comprise undesired hydrocarbons, often C4 to C20 oligomers and/or unsaturated hydrocarbons, produced as byproducts from reactions such as C2 and C3 acetylene polymerisation during ethylene production.

Green oil fouling, i.e. deposits of green oil in the ethylene production system, can lead to deactivated catalysts, contaminated furnaces, contaminated product streams, reduced run length, and reduced furnace efficiency which can cause expensive shutdowns of the production plant in order to remove the green oil fouling.

In the present ethylene production method, green oil fouling is significantly reduced by utilising a recycle propane heavies removal unit. Reference to this unit herein includes a recycle propane heavies removal unit which is or comprises a heavies removal tower. This unit enables green oil to be efficiently separated from propane streams to prevent it from re-entering other parts of the system, such as cracking furnaces. Green oil can be recovered for use in heavier oil fractions and gasoline whilst propane can be recycled for further cracking to olefins. In this way the waste in the system is reduced and costly shutdowns of the plant for removing green oil related fouling are avoided.

In the present method, a feedstock undergoes a cracking process to convert it into a product stream comprising ethylene, propylene and propane. Generally, the feedstock comprises hydrocarbons, typically liquid feedstocks such as naphtha or liquefied petroleum gas (LPG), or gaseous feedstocks, typically ethane derived from natural gas.

During the cracking process, longer hydrocarbons in the feedstock are cracked into shorter hydrocarbons such as ethylene, propylene and propane. The cracking process is typically conducted at temperature of about 750°C to about 900°C or about 800°C to about 850°C in a fired heater generally termed a pyrolysis furnace. The desired product components may typically be generated within fractions of a second inside the radiant coils of the furnace.

Cracked gases from the pyrolysis furnace are then quenched and cooled to prevent subsequent undesirable side reactions. The product stream is separated into at least an ethylene enriched stream and a propylene enriched stream comprising propane. There may also be other valuable streams in the cracked effluent.

In certain embodiments, the term ethylene enriched stream is intended to describe a stream typically comprising about 65 mol% to about 90 mol% of ethylene based on the total ethylene enriched stream. The ethylene enriched stream also typically comprises ethane and C3 hydrocarbon components. Herein, the term "enriched" generally refers to the proportional amount of a component having been increased and thus takes its usual meaning in the art.

In certain embodiments, the term propylene enriched stream is intended to describe a stream typically comprising at least about 90 mol% propylene based on the total propylene enriched stream. The propylene enriched stream also typically comprises propane and C4+ green oil components, typically generated in the MAPD hydrogenation reaction.

A C2 splitting unit is typically used to separate the ethylene in the ethylene enriched stream from any ethane that is present using fractionation. This separation forms a top product enriched in ethylene and a bottoms product enriched in ethane, often referred to as recycle ethane. Typically, the top product enriched in ethylene contains more than about 90 mol% ethylene, or more than about 95 mol ethylene, or more than about 99 mol% ethylene, or more than about 99.9 mol% ethylene, or about 99.95 mol% ethylene. The top product enriched in ethylene may be a suitable feedstock for downstream polymer units. The separated ethane may be recycled for cracking.

A C3 splitting unit is typically used to separate the propylene in the propylene and propane enriched stream from the propane using fractionation. This separation forms a top product enriched in propylene and a bottoms product enriched in propane, often referred to as recycle propane, and further comprising green oil impurities. Typically, the amount of propylene in the top product enriched in propylene is more than about 90 mol%, or more than about 95 mol%, or more than about 99 mol%, or about 99.5 mol% propylene based on the total top product enriched in propylene. The top product enriched in propylene may be used for feedstock in downstream polymer units.

Typically, the amount of green oil in the bottoms product is at least about 2% by weight of the bottoms product. More typically, the amount of green oil in the bottoms product is from about 2% to about 10% by weight of the bottoms product, or about 2% to about 8% by weight of the bottoms product.

The recycle propane from the C3 splitting unit is separated from the green oil impurity in the bottoms product by fractionation in a heavies removal unit to produce a bottoms product enriched in green oil and a top product enriched in propane. Figure 1 shows an exemplary process for removing green oil impurities from a propane recycle stream according to the method described herein. A propane recycle stream (1) enters the heavies removal tower (2) for fractionation from green oil impurities. A reboiler exchanger (3) transfers heat to the heavies removal tower (2) for fractionation. Vapourised propane (4) exits the heavies removal tower from the top and a bottom product enriched in green oil (5) exits the heavies removal tower at the bottom. A temperature controller (TC) or reboiler outlet temperature controller is situated between the heavies removal tower (2) and the reboiler exchanger (3).

As shown in figure 1, the recycle propane stream (1) enters the heavies removal tower (2) for fractionation. Typically, the inlet temperature of the heavies removal unit is about 35°C to about 75 °C, or about 40°C to about 55°C, or about 40°C to about 50°C. The inlet temperature may be determined by the equilibrium conditions of the feed stream at the selected operating pressure. Typically, the inlet pressure of the heavies removal unit is from about 1000 kPa(g) to about 1500 kPa(g), optionally from about 1000 kPa(g) to about 1300 kPa(g), optionally from about 1100 kPa(g) to about 1300 kPa(g), optionally from about 1225 kPa(g) to about 1275 kPa(g). The pressure will usually be set by the cracking furnace feed pressure requirement.

The heavies removal unit is re-boiled with steam at a pressure of about 150 kPa(g) to about 250 kPa(g) or about 200 kPa(g) using a reboiler exchanger (3). The temperature in the reboiler exchanger may be about 100°C to about 150°C, or about 120°C to about 140°C. This re-boiling causes the recycle propane to be vapourised and rise to the top of the heavies removal unit. The re-boiling process typically occurs at a temperature of about 80-120 °C, or about 100 °C. The re-boiling process typically occurs at a pressure of from about 1000 to about 1500 kPa(g), or from about 1000 to about 1300 kPa(g). The skilled person will understand that the pressure of the reboiler exchanger will depend on the requirements of the cracking furnace feed and so will be able to adjust the pressure accordingly.

The unit overhead containing the vapourised propane (4) is typically sent to a recycle furnace for cracking. Typically, the outlet temperature of the propane from the top of the heavies removal unit is from about 25°C to about 100°C, optionally from about 25°C to about 50°C, optionally from about 40°C to about 50 °C. The skilled person will understand that the outlet temperature of the pressure may vary depending on the desired operating pressure of the heavies removal unit.

The green oil enriched bottom product remains at the bottom of the heavies removal unit. The bottom product enriched in green oil can be recovered into heavier gasoline and oil fractions (5). Typically, the outlet flow rate of the bottom product enriched in green oil from the bottom of the heavies removal unit is about 5 to about 20% of the heavies removal unit feed rate (1), depending on observed green oil content in the feed stream.

Removal of green oil and other impurities from the recycle propane is important because the vapourised recycle propane is typically used in for further cracking. Removing the green oil from the propane prevents green oil from entering the cracking furnace or recycle furnace from the recycle propane stream, which can lead to reduced furnace efficiency as well as secondary transfer heat line exchanger (TLE) fouling.

Control of the vaporisation of recycle propane can be accomplished by controlling the ratio of vapourised recycle propane (4) to the total recycle propane stream flow (1). The ratio control set point is adjustable using a ratio controller and can be set based on the expected green oil content in the recycle propane stream. Typically, the amount of green oil expected in the recycle propane stream is at least about 2% by weight, or at least about 5% by weight, or at least about 8% by weight, or about 10% by weight. The amount of green oil expected in the recycle propane stream may be about 2% to about 15% by weight, or about 8% to about 15% by weight. Depending on the observed green oil content in the recycle propane stream the ratio control set point may be set at about 80% to about 95%, or about 85% to about 95% or about 85% to about 90%. It is also desirable to recover about 92-98 wt% of propane from the propane recycle stream (1). Typically, more than about 80%, or more than about 90% of the propane from the propane recycle stream may be recovered. More typically, about 90% to about 98%, or about 92% to about 98% of the propane from the propane recycle stream may be recovered. As such, the ratio control can typically be set around 88% by weight but can be varied within a range from range of 80% to 95%, depending on observed green oil content in the feed stream.

A reboiler outlet temperature controller (TC) can be used to override the ratio controller to prevent overheating and fouling the reboiler exchanger. In this manner green oil contained in the propane recycle stream is continuously withdrawn, preventing the green oil from entering the cracking furnace where it can cause reduced furnace efficiency as well as secondary transfer heat line exchanger (TLE) fouling. The reboiler outlet temperature controller (TC) override setpoint may be set to limit the temperature at the reboiler outlet stream (6) to a maximum temperature of about 100°C to about 150°C, or about 100°C to about 125°C, or about 100°C to about 110°C to prevent fouling of the reboiler exchanger (3) and heavies removal tower (2). If the temperature of the reboiler exchanger reaches above about 100°C, the ratio control set point may be reduced to lower the temperature of the reboiler exchanger back to a desired range.

Also provided herein is a system for producing ethylene. The system may comprise a reactor or cracking furnace for cracking a feedstock to convert it into a product stream comprising ethylene and propane.

The system may also comprise a separation unit for separating the cracking product stream into several streams, including at least an ethylene enriched stream and a propylene enriched stream which further comprises propane. The separation unit may comprise one or more processing units such as units for fractionation, reactors, dehydrators, and/or heat transfer equipment in which products of the cracking product stream are processed into desired polymer units, such as polyethylene.

The system may comprise a C3 splitting unit, typically containing distillation columns, for separating the propylene enriched stream comprising propane to form a top product enriched in propylene and a bottoms product enriched in propane and further comprising a green oil impurity. The C3 splitting unit may comprise distillation column(s) containing mass transfer plates to obtain the necessary polymer grade quality for the propylene product.

The system may also comprise a heavies removal unit for separating the propane from the green oil impurity in the bottoms product (obtained from the C3 splitting unit) to produce a bottoms product enriched in green oil and a top product enriched in propane. The heavies removal unit may comprise a packed bed column providing a plurality of stages of fractionation of the propane and green oil impurity containing bottoms product. For example, the heavies removal unit may comprise a packed bed column for two or more stages of fractionation, or three or more stages of fractionation. Optionally, the packed bed column is catalyst-free. The heavier green oil components will fractionate to the bottom of the heavies removal unit and the lighter propane will fractionate to the top of the heavies removal unit as an overhead product. The green oil enriched bottoms product may be drawn off from the heavies removal unit. A flow controller (FC) may be used to tailor the amount of green oil enriched bottom product contained at the bottom of the heavies removal unit by controlling the rate at which the green oil enriched bottoms product is drawn off. The set point of the flow controller may be reset by a heavies removal unit controller to provide further control of the amount of green oil enriched bottoms product contained at the bottom of the heavies removal unit at any one time.

The height of the packed bed column may be tailored depending on the desired number of stages of fractionation. For example, a packed bed column with a height of about 6 m T/T may provide three or more stages of fractionation. The packed bed may contain conventional random packing to provide mass transfer. The packed bed may comprise a liquid distribution device is provided above the packed bed to efficiently and evenly distribute recycle propane liquid to the packing over a range of flow rates. Typically the flow rates of the recycle propane in the packed bed column may be from about 70% to about 110% of the design flow rate. The liquid distributor is optionally at the top of the packed bed, i.e. at the top of the heavies removal unit. The heavies removal unit may also comprise a vapour distributor to efficiently and evenly distribute the propane vapour generated in the reboiler exchanger (3) to the packing over a range of flow rates. The vapour distributor may optionally be located at the bottom of the of the packed bed, i.e. at the bottom of the heavies removal unit.

The system may further comprise a ratio controller for adjusting the ratio control set point based on the expected green oil content in the recycle propane stream. The ratio controller may generally be used to control the ratio of vapourised recycle propane (4) to the total recycle propane stream flow (1).

The system may further comprise a temperature controller (TC), optionally located between the heavies removal tower (2) and the reboiler exchanger (3). The temperature controller, or reboiler outlet temperature controller, can be configured to override the ratio controller to prevent overheating and fouling the reboiler exchanger.

The heavies removal unit may comprise instrumentation to prevent liquid green oil carryover into the overhead propane product stream. The instrumentation may be located at the bottom of the heavies removal unit.

The heavies removal unit may comprise a mist eliminator to prevent carryover of liquid mists containing green oil. The mist eliminator may optionally be located at the top of the heavies removal unit.

The system may further comprise quench units for quenching and cooling cracked gases.

The system may further comprise one or more compression units which may be used to compress the cooled cracked gases. Between compression units, there may be one or more scrubbing units to remove gases such as H₂S and CO₂ from gas streams.

The system may comprise one or more heat exchangers for heating or cooling of gas streams. The heat exchangers may optionally include molecular sieves to dry gas streams. The skilled person would be able to select suitable molecular sieves. An example of a heat exchange is the reboiler exchanger which is typically connected to the heavies removal unit. The reboiler exchanger transfers heat to the heavies removal unit for the fractionation of propane and green oil described herein.

It will be understood that the description of preferred embodiments herein is given by way of example only and that various modifications may be made by those skilled in the art. What has been described above includes examples of one or more embodiments. It is, of course, not possible to describe every conceivable modification and alteration of the above process and apparatus for purposes of describing the aforementioned aspects, but one of ordinary skill in the art can recognize that many further modifications and permutations of various aspects are possible. Accordingly, the described aspects are intended to embrace all such alterations, modifications, and variations that fall within the scope of the appended claims.

The following numbered embodiments are provided as a part of the disclosure:
1. An ethylene production method, wherein the method comprises:
   a) cracking a feedstock to convert it into a product stream comprising ethylene, propylene and propane;
   b) separating the product stream into an ethylene enriched stream and a propylene enriched stream comprising propane;
   c) separating the propylene enriched stream to form a top product enriched in propylene and a bottoms product enriched in propane and further comprising a green oil impurity; and
   d) separating the propane from the green oil impurity in the bottoms product by fractionation in a heavies removal unit to produce a bottoms product enriched in green oil and a top product enriched in propane.
2. The method of embodiment 1, wherein the fractionation in the heavies removal unit comprises a step of vaporising the propane using a reboiler exchanger, optionally utilizing steam as a heating medium at a pressure of about 150 kPa(g) to about 250 kPa(g), and/or about 100°C to about 150°C.
3. The method of embodiment 2, further comprising controlling vaporisation of the propane by controlling and/or adjusting the ratio of the amount of top product enriched in propane produced in the heavies removal unit to the amount of bottoms product enriched in propane and further comprising the green oil impurity that is fed to the heavies removal unit, optionally wherein the controlling is performed by a ratio controller.
4. The method of embodiment 3, further comprising monitoring the temperature of a reboiler outlet stream and adjusting the ratio of the top product to the bottoms product when the reboiler outlet stream temperature exceeds a temperature threshold, optionally wherein the adjustment is performed by a reboiler outlet temperature controller.
5. The method of any preceding embodiment, wherein the heavies removal unit has an inlet pressure of from about 1000 kPa(g) to about 1500 kPa(g), optionally about 1000 kPa(g) to about 1300 kPa(g).
6. The method of any preceding embodiment, wherein the heavies removal unit has an inlet temperature of from about 25°C to about 90°C, optionally from about 35°C to about 75°C, optionally from about 40°C to about 50°C.
7. The method of any preceding embodiment, wherein the green oil impurity is present in the bottoms product enriched in propane and further comprising a green oil impurity in an amount of at least about 2% by weight.
8. The method of any preceding embodiment, wherein the top product enriched in propane contains green oil impurity in an amount of less than about 1% by weight.
9. The method of any preceding embodiment, wherein the green oil impurity is continuously removed from the heavies removal unit.
10. The method of any preceding embodiment further comprising the step of recycling the top product enriched in propane for cracking.
11. The method of any preceding embodiment, wherein the heavies removal unit comprises a packed bed column and/or is catalyst-free.
12. The method of any preceding embodiment, wherein the bottoms product enriched in propane and further comprising a green oil impurity is in the liquid phase.
13. The method of any preceding embodiment, further comprising the step of recovering the bottoms product enriched in green oil and mixing it with gasoline and/or oil product streams.
14. A system for producing ethylene, wherein the system comprises:
   a reactor for cracking a feedstock to convert it into a product stream comprising ethylene, propylene and propane;
   a separation unit for separating the product stream into an ethylene enriched stream and a propylene enriched stream which further comprises propane;
   a C3 splitting unit for separating the propylene enriched stream to form a top product enriched in propylene and a bottoms product enriched in propane and further comprising a green oil impurity; and a heavies removal unit for separating the propane from the green oil impurity in the bottoms product by fractionation to produce a bottoms product enriched in green oil and a top product enriched in propane.
15. The system of embodiment 14, wherein the heavies removal unit comprises a packed bed column, optionally configured to provide a plurality of fractionation stages.
16. The system of embodiment 14 or 15, wherein the heavies removal unit is catalyst-free.
17. The system of any of embodiments 14 to 16, further comprising a reboiler exchanger configured to transfer heat to the heavies removal unit.
18. The system of any of embodiments 14 to 17, wherein the system comprises a ratio controller between the heavies removal unit product and C3 splitting unit, optionally wherein the ratio controller is configured to adjust the ratio of the amount of top product enriched in propane produced in the heavies removal unit to the amount of bottoms product enriched in propane and further comprising the green oil impurity that is fed to the heavies removal unit.
19. The system of any of embodiments 14 to 18, further comprising an outlet temperature controller between the reboiler exchanger and the heavies removal unit, optionally wherein the outlet temperature controller is configured to adjust the ratio of the top product to the bottoms product when the reboiler outlet stream temperature is determined to exceed a temperature threshold.
20. A method of removing a green oil impurity from a propane stream produced in an ethylene production method, wherein the method comprises:
   separating the propane in the propane stream from the green oil impurity by fractionation in a heavies removal unit to produce a bottoms product enriched in green oil and a top product enriched in propane.

## Claims

1. An ethylene production method, wherein the method comprises:
a) cracking a feedstock to convert it into a product stream comprising ethylene, propylene and propane;
b) separating the product stream into an ethylene enriched stream and a propylene enriched stream comprising propane;
c) separating the propylene enriched stream to form a top product enriched in propylene and a bottoms product enriched in propane and further comprising a green oil impurity; and
d) separating the propane from the green oil impurity in the bottoms product by fractionation in a heavies removal unit to produce a bottoms product enriched in green oil and a top product enriched in propane.

2. The method of claim 1, wherein the fractionation in the heavies removal unit comprises a step of vaporising the propane using a reboiler exchanger, optionally utilizing steam as a heating medium at a pressure of about 150 kPa(g) to about 250 kPa(g), and/or about 100°C to about 150°C.

3. The method of claim 2, further comprising controlling vaporisation of the propane by controlling and/or adjusting the ratio of the amount of top product enriched in propane produced in the heavies removal unit to the amount of bottoms product enriched in propane and further comprising the green oil impurity that is fed to the heavies removal unit, optionally wherein the controlling is performed by a ratio controller.

4. The method of claim 3, further comprising monitoring the temperature of a reboiler outlet stream and adjusting the ratio of the top product to the bottoms product when the reboiler outlet stream temperature exceeds a temperature threshold, optionally wherein the adjustment is performed by a reboiler outlet temperature controller.

5. The method of any preceding claim, wherein the heavies removal unit has an inlet pressure of from about 1000 kPa(g) to about 1500 kPa(g), optionally about 1000 kPa(g) to about 1300 kPa(g).

6. The method of any preceding claim, wherein the heavies removal unit has an inlet temperature of from about 25°C to about 90°C, optionally from about 35°C to about 75°C, optionally from about 40°C to about 50°C.

7. The method of any preceding claim, wherein the green oil impurity is present in the bottoms product enriched in propane and further comprising a green oil impurity in an amount of at least about 2% by weight; and/or
wherein the top product enriched in propane contains green oil impurity in an amount of less than about 1% by weight.

8. The method of any preceding claim, wherein the heavies removal unit comprises a packed bed column and/or is catalyst-free.

9. The method of any preceding claim, wherein the bottoms product enriched in propane and further comprising a green oil impurity is in the liquid phase.

10. The method of any preceding claim, further comprising the step of recovering the bottoms product enriched in green oil and mixing it with gasoline and/or oil product streams.

11. A system for producing ethylene, wherein the system comprises:
a reactor for cracking a feedstock to convert it into a product stream comprising ethylene, propylene and propane;
a separation unit for separating the product stream into an ethylene enriched stream and a propylene enriched stream which further comprises propane;
a C3 splitting unit for separating the propylene enriched stream to form a top product enriched in propylene and a bottoms product enriched in propane and further comprising a green oil impurity; and a heavies removal unit for separating the propane from the green oil impurity in the bottoms product by fractionation to produce a bottoms product enriched in green oil and a top product enriched in propane.

12. The system of claim 11, wherein the heavies removal unit comprises a packed bed column, optionally configured to provide a plurality of fractionation stages; and/or wherein the heavies removal unit is catalyst-free.

13. The system of claims 11 or 12, further comprising a reboiler exchanger configured to transfer heat to the heavies removal unit.

14. The system of any of claims 11 to 13, wherein the system comprises a ratio controller between the heavies removal unit product and C3 splitting unit, optionally wherein the ratio controller is configured to adjust the ratio of the amount of top product enriched in propane produced in the heavies removal unit to the amount of bottoms product enriched in propane and further comprising the green oil impurity that is fed to the heavies removal unit.

15. The system of any of claims 11 to 14, further comprising an outlet temperature controller between the reboiler exchanger and the heavies removal unit, optionally wherein the outlet temperature controller is configured to adjust the ratio of the top product to the bottoms product when the reboiler outlet stream temperature is determined to exceed a temperature threshold.

## Amended claims

### Amended claims in accordance with Rule 137(2) EPC.

1. An ethylene production method, wherein the method comprises:
a) cracking a feedstock to convert it into a product stream comprising ethylene, propylene and propane;
b) separating the product stream into an ethylene enriched stream and a propylene enriched stream comprising propane;
c) separating the propylene enriched stream to form a top product enriched in propylene and a bottoms product enriched in propane and further comprising a green oil impurity comprising a mixture of C4 to C20 oligomers and/or unsaturated hydrocarbons; and
d) separating the propane from the green oil impurity in the bottoms product by fractionation in a heavies removal unit to produce a bottoms product enriched in green oil and a top product enriched in propane.

2. The method of claim 1, wherein the fractionation in the heavies removal unit comprises a step of vaporising the propane using a reboiler exchanger, optionally utilizing steam as a heating medium at a pressure of about 150 kPa(g) to about 250 kPa(g), and/or about 100°C to about 150°C.

3. The method of claim 2, further comprising controlling vaporisation of the propane by controlling and/or adjusting the ratio of the amount of top product enriched in propane produced in the heavies removal unit to the amount of bottoms product enriched in propane and further comprising the green oil impurity that is fed to the heavies removal unit, optionally wherein the controlling is performed by a ratio controller.

4. The method of claim 3, further comprising monitoring the temperature of a reboiler outlet stream and adjusting the ratio of the top product to the bottoms product when the reboiler outlet stream temperature exceeds a temperature threshold, optionally wherein the adjustment is performed by a reboiler outlet temperature controller.

5. The method of any preceding claim, wherein the heavies removal unit has an inlet pressure of from about 1000 kPa(g) to about 1500 kPa(g), optionally about 1000 kPa(g) to about 1300 kPa(g).

6. The method of any preceding claim, wherein the heavies removal unit has an inlet temperature of from about 25°C to about 90°C, optionally from about 35°C to about 75°C, optionally from about 40°C to about 50°C.

7. The method of any preceding claim, wherein the green oil impurity is present in the bottoms product enriched in propane and further comprising a green oil impurity in an amount of at least about 2% by weight; and/or
wherein the top product enriched in propane contains green oil impurity in an amount of less than about 1% by weight.

8. The method of any preceding claim, wherein the heavies removal unit comprises a packed bed column and/or is catalyst-free.

9. The method of any preceding claim, wherein the bottoms product enriched in propane and further comprising a green oil impurity is in the liquid phase.

10. The method of any preceding claim, further comprising the step of recovering the bottoms product enriched in green oil and mixing it with gasoline and/or oil product streams.

11. A system for producing ethylene, wherein the system comprises:
a reactor for cracking a feedstock to convert it into a product stream comprising ethylene, propylene and propane;
a separation unit for separating the product stream into an ethylene enriched stream and a propylene enriched stream which further comprises propane;
a C3 splitting unit for separating the propylene enriched stream to form a top product enriched in propylene and a bottoms product enriched in propane and further comprising a green oil impurity comprising a mixture of C4 to C20 oligomers and/or unsaturated hydrocarbons;
a heavies removal unit for separating the propane from the green oil impurity in the bottoms product by fractionation to produce a bottoms product enriched in green oil and a top product enriched in propane; and
a ratio controller between the heavies removal unit product and C3 splitting unit, and/or an outlet temperature controller between the reboiler exchanger and the heavies removal unit.

12. The system of claim 11, wherein the heavies removal unit comprises a packed bed column, optionally configured to provide a plurality of fractionation stages; and/or wherein the heavies removal unit is catalyst-free.

13. The system of claims 11 or 12, further comprising a reboiler exchanger configured to transfer heat to the heavies removal unit.

14. The system of any of claims 11 to 13, wherein the ratio controller is configured to adjust the ratio of the amount of top product enriched in propane produced in the heavies removal unit to the amount of bottoms product enriched in propane and further comprising the green oil impurity that is fed to the heavies removal unit.

15. The system of any of claims 11 to 14, wherein the outlet temperature controller is configured to adjust the ratio of the top product to the bottoms product when the reboiler outlet stream temperature is determined to exceed a temperature threshold.
